(19) Europäisches Patentamt
European Patent Office
Office européen des brevets

(11) Veröffentlichungsnummer: **0 104 566**
**B1**

(12) **EUROPÄISCHE PATENTSCHRIFT**

(45) Veröffentlichungstag der Patentschrift: **25.02.87**

(21) Anmeldenummer: **83109230.9**

(22) Anmeldetag: **17.09.83**

(51) Int. Cl.⁴: **C 07 D 471/04** // A61K31/55 ,(C07D471/04, 243:00, 221:00)

(54) **Verfahren zur Herstellung von 5,11-Dihydro-11-((4-methyl-1-piperazinyl)-acetyl)-6H-pyrido(2,3-b)(1,4)benzodiazepin-6-on.**

(30) Priorität: **28.09.82 DE 3235795**

(43) Veröffentlichungstag der Anmeldung: **04.04.84 Patentblatt 84/14**

(45) Bekanntmachung des Hinweises auf die Patenterteilung: **25.02.87 Patentblatt 87/9**

(84) Benannte Vertragsstaaten: **AT CH DE IT LI NL SE**

(56) Entgegenhaltungen:
**FR-A-2 478 099**

**CHEMICAL ABSTRACTS, Band 96, Nr. 9, 01-03-1982, Seite 619, Nr. 69054v, COLUMBUS, OHIO (US)**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Eberlein, Wolfgang, Dr. Dipl.- Chem., Obere Au 6, D-7950 Biberach 1 (DE)**
Erfinder: **Breuer, Gerhard, Händelstrasse 14, D-7950 Biberach (DE)**

**EP 0 104 566 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein neues Verfahren zur Herstellung von 5,11-Dihydro-11-[(4-methyl-1-piperazinyl)-acetyl]-6H-pyrido[2,3-b][1,4]benzodiazepin-6-on (Pirenzepin) bzw. seiner Salze mit anorganischen oder organischen Säuren. Diese Verbindung hat aufgrund ihrer hervorragenden Antiulcus-Wirkung große therapeutische Bedeutung erlangt.

Die Herstellung von Pirenzepin wird in der DE-PS 1 795 183 als ein Zweistufen-Verfahren, ausgehend von 5,11-Dihydro-6H-pyrido[2,3-b][1,4]benzodiazepin-6-on, beschrieben. Die Ausgangsverbindung wird hierbei zuerst mit einem Halogenacetylhalogenid in ein Zwischenprodukt, 11-Halogenacetyl 5,11-dihydro-6H-pyrido[2,3-b][1,4]benzodiazepin-6-on, übergeführt, anschließend wird dieses Zwischenprodukt mit N-Methylpiperazin zum Endprodukt umgesetzt.

Es war bereits wiederholt versucht worden die Seitenkette in einer Stufe einzuführen (siehe beispielsweise EP-A-0 022 144 oder DE-A-31 09 769), die dort postulierten Verfahren sind jedoch in der Praxis völlig undurchführbar.

Es wurde nun gefunden, daß man Pirenzepin aus 5,11-Dihydro-6H-pyrido[2,3-b][1,4]benzodiazepin-6-on I in guten Ausbeuten auf einfache Weise in einer einstufigen Reaktion erhalten kann, wenn man es zuerst in das Di-Lithiumsalz II überführt und letzteres anschließend mit einem (4-Methyl-1-iperazinyl)-essigsäureester III nach dem folgenden Reaktionsschema zum pirenzepin IV umsetzt:

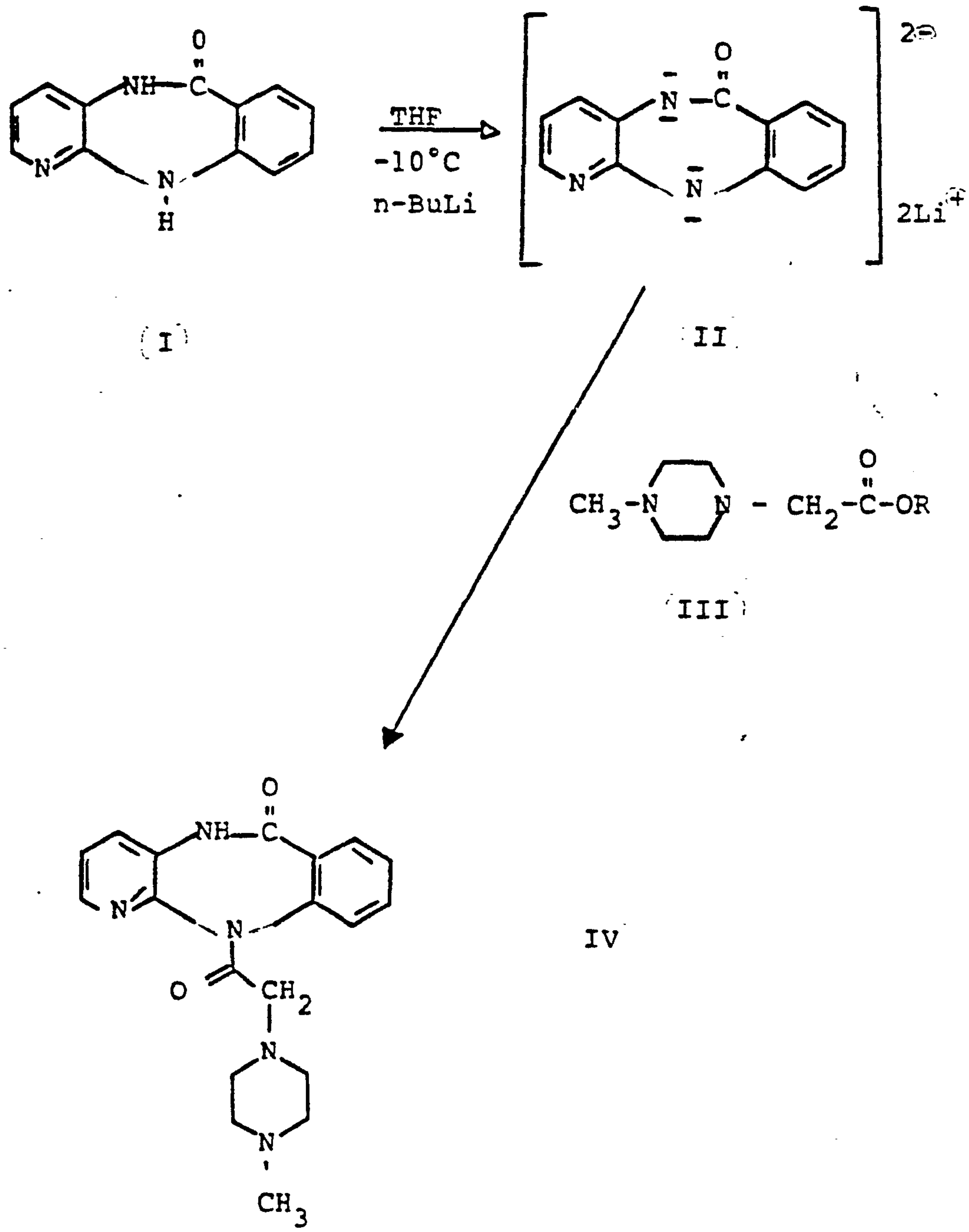


(I) II III IV

In dem (4-Methyl-1-piperazinyl)-essigsäureester der allgemeinen Formel III bedeutet R einen Alkylrest mit 1 bis 10 Kohlenstoffatomen, vorzugsweise 1 bis 6 Kohlenstoffatomen oder einen Phenyl- oder Phenylalkylrest mit 1 bis 3 C-Atomen im Alkylteil.

Die Überführung des 5,11-Dihydro-6H-pyrido[2,3-b][1,4]benzo-diazepin-6-ons (Formel I) in das Lithiumsalz der Formel II gelingt mit Lithiumalkylen, insbesondere aber mit n-Butyllithium, n-Butyllithium in Gegenwart von Tetramethylethylendiamin, tertiär-Butyllithium, Lithiumdiisopropylamid oder Lithiumdicyclohexylamid oder mit Lithiumarylen, z. B. mit Lithiumphenyl. Die Überführung in das Lithiumsalz und die weitere Umsetzung zum

Pirenzepin erfolgt in einem organischen Lösungsmittel bei Temperaturen zwischen -60°C und 0°C, vorzugswsise aber bei -10°C. Als organische Lösungsmittel dienen solche, die für Umsetzungen mit Lithiumalkylen oder Lithiumarylen gebräuchlich sind; besonders vorteilhaft ist die Verwendung von Tetrahydrofuran oder Ethern, wie Diethylether, von aliphatischen Kohlenwasserstoffen, wie Hexan oder von Gemischen hiervon, gegebenenfalls auch in Gegenwart von Hexamethylphosphoramid als Cosolvens. Kurze Zeit nach der Beendigung der Zugabe des Lithiumalkyls oder -aryls gibt man die stöchiometrische Menge oder einen leichten Überschuß an (4-Methyl-1-piperazinyl)-essigsäureester der allgemeinen Formel III hinzu und läßt das Reaktionsgemisch zur Vervollständigung der Reaktion langsam, z. B. innerhalb von 2 Stunden, auf Raumtemperatur kommen. Das gebildete Pirenzepin der Formel IV wird nach üblichen Methoden aus dem Reaktionsgemisch isoliert und man erhält die freie Verbindung, die anschließend gewünschtenfalls in ihre Salze überführt werden kann.

Der Verlauf der Reaktion der Verbindung der Formel I mit dem Lithiumalkyl oder -aryl zu der Lithium-Zwischenverbindung der Formel II war nicht vorhersehbar, da bekanntlich die Umsetzung von Pyridinen und kondensierten Pyridinen mit metallorganischen Verbindungen vorzugsweise zu alkylsubstituierten Pyridinderivaten führt [vgl. H.W. Gschwend und H.R. Rodriguez, Heteroatom-Facilitated Lithiations, Organic Reactions, 26, 27 (1979)].

Die folgenden Beispiele sollen das Wesen der Erfindung näher erläutern:

**Beispiel 1**

**5,11-Dihydro-11-[(4-methyl-1-piperazinl)acetyl] 6H-pyrido [2,3-b][1,4-]benzodiazepin-6-on**

Zu einer Suspension von 21 g (0,1 Mol) 5,11-Dihydro-6H-pyri-do[2,3-b][1,4]benzodiazepin-6-on in 400 ml Tetrahydrofuran werden unter Rühren bei -10°C 200 ml einer 1,5 molaren Lösung von n-Butyllithium in Hexan langsam zugetropft. Nach Beendigung der Zugabe wird das Gemisch 30 Minuten bei -10°C nachgerührt. Anschließend tropft man eine Lösung von 20,4 g (0,11 Mol) (4-Methyl-1-piperazinyl)-essigsäureethylester in 100 ml absolutem Tetrahydrofuran zu. Man läßt den Ansatz auf Raumtemperatur kommen und rührt noch zwei Stunden weiter. Danach wird das Reaktionsgemisch mit Essigsäureethylester verdünnt und mit 250 ml 10 %iger Salzsäure versetzt. Der anfallende Niederschlag wird abgesaugt. Nach Abtrennung der organischen Phase wird die wäßrige Phase durch Zugabe von festem Kaliumcarbonat alkalisch gestellt und mehrmals mit Chloroform extrahiert. Man filtriert über Aktivkohle und dampft am Rotationsverdampfer zur Trockne ein. Das Rohprodukt wird säulenchromatographisch an Kieselgel unter Verwendung von Essigsäureethylester/Methanol im Volumenverhältnis 9:1 gereinigt. Man erhält 21,1 g (60 % der Theorie) eines farblosen Produkts, das nach dem Umkristallisieren aus Methanol bei 222-225°C schmilzt und nach Dünnschichtchromatogramm, IR- und NMR-Spektren mit der im DE-PS 1 795 183, Beispiel 7, beschriebenen Verbindung völlig identisch ist.

**Beispiel 2**

**5,11-Dihydro-11-[(4-methyl-1-piperazinyl)acetyl]-6H-pyrido-[2,3-b][1,4]benzodiazepin-6-on**

Eine Suspension von 21 g (0,1 Mol) 5,11-Dihydro-6H-pyrido-[2,3-b][1,4]benzodiazepin-6-on in 400 ml Tetrahydrofuran wird unter Stickstoff auf -60°C abgekühlt und anschließend in eine ebenfalls auf -60°C abgekühlte Lösung von 200 ml Lithiumdiisopropylamid, hergestellt aus 20,2 g Diisopropylamin und 153,8 ml einer 1,3 molaren Lösung von n-Butyllithium in Hexan, und 50 ml Hexamethylphosphoramid eingetropft. Nach 30-minütigem Rühren bei der gleichen Temperatur tropft man eine Lösung von 24,7 g (0,10 Mol) (4-Methyl-1-piperazinyl)-essigsäurebenzylester in 100 ml Tetrahydrofuran zu. Man setzt das Rühren noch zwei Stunden fort und läßt dann auf Raumtemperatur kommen. Die Aufarbeitung des Reaktionsansatzes erfolgt analog der im Beispiel 1 beschriebenen Methods. Die Reinigung des Rohproduktes erfolgt durch Chromatographie an Kieselgel unter Verwendung von Essigsäureethylester/Methanol im Volumenverhältnis 3:1. Das anfallende farblose Produkt ist nach Dünnschichtchromatogramm, IR- und NMR-Spektren mit der nach Beispiel 1 beschriebenen Verbindung völlig identisch.

Ausbeute: 21,8 g (62 % der Theorie).

**Beispiel 3**

**5,11-Dihydro-11-[(4-methyl-1-piperazinyl)acetyl]-6H-pyrido-[2,3-b][1, 4]benzodiazepin-6-on**

Analog Beispiel 1 werden unter Verwendung von 17,2 g (0,10 Mol) 4-Methyl-1-piperazinylessigsäuremethylester 20,2 g (57 % der Theorie) der gewünschten Verbindung erhalten.

Vergleichbare Ergebnisse erhält man bei der Verwendung des (4-Methyl-1-piperazinyl)-essigsäure-n-propyl- oder -2-phenylethylesters.

**Beispiel 4**

**5,11-Dihydro-11-[(4-methyl-1-piperazinyl)acetyl]-6H-pyrido-[2,3-b][1,4]benzodiazepin-6-on**

Analog Beispiel 1 werden 21 g (0,1 Mol) 5,11-Dihydro-6H-py-rido[2,3-b][1,4]benzodiazepin-6-on mit 36 g (0,15 Mol) (4-Methyl-1-piperazinyl)-essigsäurehexylester umgesetzt. Nach der säulenchromatographischen Reinigung erhält man 11,9 g (34,7 % der Theorie) eines farblosen Produktes, welches nach dem Umkristallisieren aus Methanol bei 222-224° C schmilzt und nach den spektroskopischen Daten völlig identisch ist mit der nach Beispiel 1 beschriebenen Verbindung.

**Beispiel 5**

**5,11-Dihydro-11-[(4-methyl-1-piperazinyl)acetyl]-6H-pyrido-[2,3-b][1,4]benzodiazepin-6-on**

Analog Beispiel 1 werden 21 g (0,1 Mol) 5,11-Dihydro-6H-py-rido[2,3-b][1,4]benzodiazepin-6-on mit 41,4 g (0,15 Mol) (4-Methyl-1-piperazinyl)-essigsäure-3-phenylpropylester umgesetzt. Nach der säulenchromatographischen Reinigung erhält man 14,5 g (41,4 % der Theorie) eines farblosen Produktes, welches nach dem Umkristallisieren aus Methanol bei 222-225° C schmilzt und nach den spektroskopischen Daten völlig identisch ist mit der nach Beispiel 1 beschriebenen Verbindung.

**Beispiel 6**

**5,11-Dihydro-11-[(4-methyl-1-piperazinyl)acetyl]-6H-pyrido-[2,3-b][1,4]benzodiazepin-6-on**

Zu einer Suspension von 21 g (0,1 Mol) 5,11-Dihydro-6H-pyri-do[2,3-b][1,4]benzodiazepin-6-on in 400 ml Tetrahydrofuran werden unter Rühren bei -10° C 150 ml einer ca. 2 molaren Lösung von Phenyllithium in einem Gemisch von Benzol und Ether (75/25) langsam zugetropft. Nach Beendigung der Zugabe wird das Gemisch 30 Minuten bei -10° C nachgerührt. Anschließend tropft man eine Lösung von 20,4 g (0,11 Mol) (4-Methyl-1-piperazinyl)-essigsäureethylester in 100 ml absolutem Tetrahydrofuran zu. Man läßt den Ansatz auf Raumtemperatur kommen und rührt noch zwei Stunden weiter. Die Aufarbeitung erfolgt analog Beispiel 1. Man erhält 11,3 g (32,2 % der Theorie) eines farblosen Produktes, das nach den spektroskopischen Daten völlig identisch ist mit der nach Beispiel 1 erhaltenen Verbindung.

**Beispiel 7**

Vorteilhaft können anstelle von Tetrahydrofuran im Beispiel 1 auch andere organische, für die Umsetzung mit Lithiumalkylen oder Lithiumarylen gebräuchliche Lösungsmittel resp. Lösungsmittelgemische verwendet werden.

Einige Beispiele sind in der folgenden Tabelle aufgelistet: (Es gelten die im Beispiel 1 genannten Gewichtsangaben).

| Lösungsmittel | Ausbeute (g) | % der Theorie |
|---|---|---|
| Glykol-dimethylether | 15,8 | 44 |
| Dioxan/Glykoldimethylether (1:1) | 9,7 | 27 |
| Hexan/Tetramethylethylen-diamin (2:1) | 5,8 | 16 |

**Patentansprüche**

1. Verfahren zur Herstellung von 5,11-Dihydro-11-[(4-methyl-1-piperazinyl)acetyl]-6H-pyrido[2,3-b][1,4]benzodiazepin-6-on bzw. der Salze mit anorganischen oder organischen Säuren, dadurch gekennzeichnet, daß 5,11-Dihydro-6H-pyrido[2,3-b][1,4]benzodiazepin-6-on der Formel I

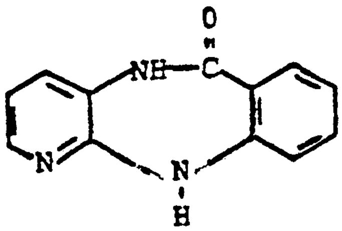

(I)

in einem für Lithiierungsreaktionen gebräuchlichen Lösungsmittel zuerst mit mindestens 2 Äquivalenten eines Lithiumalkyls oder Lithiumaryls bei Temperaturen zwischen -60 und 0° C und anschließend mit einem (4-Methyl-1-piperazinyl)-essigsäureester der allgemeinen Formel III

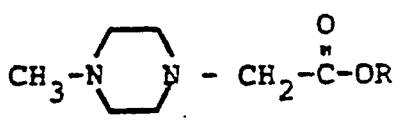

(III)

in der R einen Alkylrest mit 1 bis 10 C-Atomen, einen Phenyl- oder einen Phenylalkylrest mit 1 bis 3 C-Atomen im Alkylteil bedeutet, umgesetzt und die so erhaltene Verbindung isoliert und gegebenenfalls in ihre Salze mit anorganischen oder organischen Säuren übergeführt wird.

2. Verfahren gemäß Anspruch 1, dadurch gekennzeichnet, daß die Reaktion bei -10° C durchgeführt wird.

3. Verfahren gemäß Anspruch 1 und 2, dadurch gekennzeichnet, daß als Lithiumalkyl oder -aryl n-Butyllithium, gegebenenfalls in Gegenwart von Tetramethylethylendiamin, tertiär-Butyllithium, Lithiumdiisopropylamid, Lithiumdicyclohexylamid oder Lithiumphenyl und als Lösungsmittel Ether, wie Diethylether, Tetrahydrofuran, aliphatische Kohlenwasserstoffe, wie Hexan, Gemische dieser Lösungsmittel und, gegebenenfalls, als Cosolvens Hexamethylphosphoramid verwendet wird.

4. Verfahren gemäß Anspruch 1, 2 und 3, dadurch gekennzeichnet, daß als Ester ein solcher der allgemeinen Formel III verwendet wird, in der R eine Alkylgruppe mit 1 bis 6 Kohlenstoffatomen oder die Phenylmethyl-, Phenylethyloder Phenylpropylgruppe bedeutet.

**Claims**

1. Process for preparing 5,11-dihydro-11-[(4-methyl-1-piperazinyl)acetyl]-6H-pyrido[2,3-b][1,4]-benzodiazepin-6-one or the salts thereof with inorganic or organic acids, characterised in that 5,11-dihydro-6H-pyrido[2,3-b][l,4]benzodiazepin-6-one of formula I

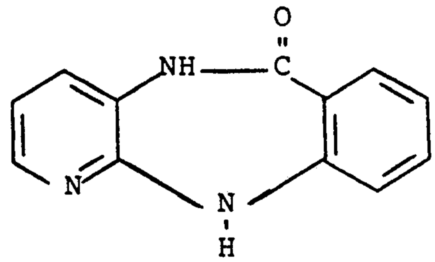

(I)

is reacted in a solvent conventionally used for lithiation reactions first with at least two equivalents of a lithium alkyl or lithium aryl at temperatures of between -60 and 0°C and subsequently with a (4-methyl-1-piperazinyl)-acetate of general formula III

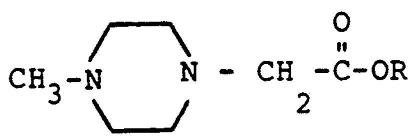

(III)

wherein R represents an alkyl group with 1 to 10 carbon atoms, a phenyl or phenylalkyl group with 1 to 3 carbon atoms in the alkyl part, and the compound thus obtained is isolated and, if desired, converted into the salts thereof with inorganic or organic acids.

2. Process as claimed in claim 1, characterised in that the reaction is carried out at -10°C.

3. Process as claimed in claims 1 and 2, characterised in that the lithium alkyl or lithium aryl used is n-butyl lithium, optionally in the presence of tetramethylethylenediamine, tertiary butyl lithium, lithium diisopropylamide, lithium dicyclohexylamide or lithium phenyl and the solvent used is an ether such as diethylether, tetrahydrofuran, aliphatic hydrocarbons such as hexane, mixtures of these solvents and, optionally, hexamethylphosphoramide is used as co-solvent.

4. Process as claimed in claims 1, 2 and 3, characterised in that the ester used is an ester of general formula III wherein R represents an alkyl group with 1 to 6 carbon atoms or a phenylmethyl, phenylethyl or phenylpropyl group.

**Revendications**

1. Procédé pour la préparation de la 5,11-di-hydro-11-((4-méthyl-1-pipérazinyl)acétyl]-6H-pyrido[2,3-b][1,4]benzodiazépine-6-one ou des sels avec des acides minéraux ou organiques, caractérisé en ce qu'on fait réagir la 5,11-dihydro-6H-pyrido[2,3-b][1,4]benzodiazé-pine-6-one de formule I

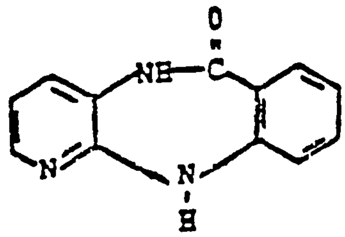

(I)

dans un solvant usuel pour les réactions avec des dérivés du lithium, d'abord avec au moins deux équivalents d'un lithium-alcoyle ou lithium-aryle à des températures entre -60 et 0° C et ensuite avec un ester de l'acide (4-méthyl-1-pipérazinyl)-acétique de formule générale III

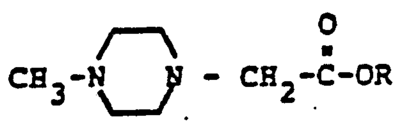

(III)

dans laquelle R représente un radical alcoyle avec 1 à 10 atomes de C, un radical phényle ou un radical phénylalcoyle avec 1 à 3 atomes de C dans la partie alcoyle et en ce qu'on isole le composé ainsi obtenu et le transforme éventuellement en ses sels avec des acides minéraux ou organiques.

2. Procédé selon la revendication 1, caractérisé en ce que la réaction est effectuée à -10° C.

3. Procédé selon la revendication 1 et 2, caractérisé en ce qu'on utilise, en tant que lithium-alcoyle ou -aryle, le n-butyllithium, éventuellement en présence de tétraméthyléthylènediamine, de tertio-butyllithium, de lithium-diisopropylamide, de lithium-dicyclohexylamide ou de lithium-phényle, et en tant que solvant des éthers comme l'éther diéthylique, le tetrahydrofuranne, des hydrocarbures aliphatiques comme l'hexane, des mélanges de ces solvants et éventuellement, en tant que co-solvant, de l'hexaméthylphosphamide.

4. Procédé selon la revendication 1, 2 et 3, caractérisé en ce que l'on utilise comme ester, un ester de formule générale III dans laquelle R représente un groupe alcoyle avec 1 à 6 atomes de carbone ou le groupe phénylméthyle, phényléthyle, ou phénylpropyle.